# EUROPÄISCHE PATENTSCHRIFT

(11) **EP 4 101 301 B1**
(45) Veröffentlichungstag und Bekanntmachung des Hinweises auf die Patenterteilung: **18.11.2026**
(21) Anmeldenummer: 21178340.2
(22) Anmeldetag: 08.06.2021
(51) Int. Cl.: A23C 9/12, A23C 21/02, C12P 19/14, C07H 1/08, C07H 3/06, C12N 9/38, C08B 37/00, C12P 19/00

(54) **VERFAHREN ZUR HERSTELLUNG VON GALACTOOLIGOSACCHARIDEN**
METHOD FOR THE PREPARATION OF GALACTOOLIGOSACCHARIDES
PROCÉDÉ DE PRODUCTION DE GALACTOOLIGOSACCHARIDES

(43) Veröffentlichungstag der Anmeldung: 14.12.2022
(73) Patentinhaber: DMK Deutsches Milchkontor GmbH, 27404 Zeven (DE)
(72) Erfinder: DÖRING, Sven-Rainer, 27404 Zeven (DE); Steffens, Marco, 27404 Elsdorf (DE)
(74) Vertreter: Fabry, Bernd

(56) Entgegenhaltungen:
- WO-A1-2008/037839
- WO-A1-2015/034356
- WO-A1-2020/117548
- CN-B- 104 004 799
- US-A1- 2020 113 200

## Beschreibung

### GEBIET DER ERFINDUNG

Die vorliegende Patentanmeldung betrifft ein Verfahren zur Herstellung von speziellen oligomeren Kohlenhydraten basierend auf Milchzucker.

### TECHNOLOGISCHER HINTERGRUND

Galactooligosaccharide (GOS) 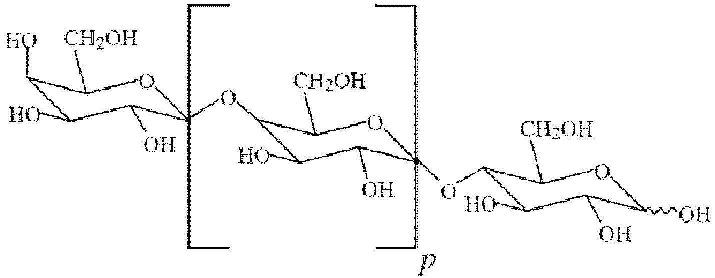 auch bekannt als Oligogalaktosyllactose, Oligogalaktose, Oligolaktose oder Transgalaktooligosaccharide (TOS), gehören zur Gruppe der Präbiotika. GOS kommt in handelsüblichen Produkten wie Nahrung für Säuglinge und Erwachsene vor.

Aufgrund der Konfiguration ihrer glykosidischen Bindungen widerstehen Galactooligosaccharide (GOS) weitgehend der Hydrolyse durch Speichel- und Darmverdauungsenzyme. Galactooligosaccharide werden daher als Präbiotika eingestuft, definiert als unverdauliche Nahrungsbestandteile, die sich vorteilhaft auf den Wirt auswirken, indem sie das Wachstum und/oder die Aktivität nützlicher Bakterien im Dickdarm stimulieren. Die erhöhte Aktivität dieser gesundheitsfördernden Bakterien führt zu einer Reihe von Effekten, sowohl direkt durch die Bakterien selbst als auch indirekt durch die organischen Säuren, die sie durch Fermentation produzieren. Beispiele für Wirkungen sind die Stimulierung der Immunfunktionen, die Aufnahme essentieller Nährstoffe, und die Synthese bestimmter Vitamine.

Galactooligosaccharide sind ein Substrat für Bakterien, wie Bifidobakterien und Laktobazillen. Studien mit Säuglingen und Erwachsenen haben gezeigt, dass mit Galakto-Oligosacchariden angereicherte Lebensmittel oder Getränke zu einer signifikanten Zunahme von Bifidobakterien führen. Diese Zucker kommen natürlich in der menschlichen Milch vor und sind als Oligosaccharide der menschlichen Milch bekannt. Beispiele sind Lakto-N-Tetraose, Lakto-N-Notetraose und Lakto-N-Fucopentaose.

Die menschliche Darmmikrobiota spielt eine Schlüsselrolle im intestinalen Immunsystem. Galacto-Oligosaccharide unterstützen die natürlichen Abwehrkräfte des menschlichen Körpers über die Darmmikroflora, indirekt, indem sie die Anzahl der Bakterien im Darm erhöhen und die Bindung oder das Überleben von *Escherichia coli, Salmonella Typhimurium* und Clostridien hemmen. GOS können das Immunsystem indirekt durch die Produktion von antimikrobiellen Substanzen positiv beeinflussen, indem sie die Vermehrung von pathogenen Bakterien reduzieren. Verstopfung ist ein potenzielles Problem, insbesondere bei Säuglingen, älteren Menschen und schwangeren Frauen. Bei Säuglingen kann die Fütterung mit Säuglingsnahrung mit Verstopfung und hartem Stuhl assoziiert sein. GOS können die Stuhlfrequenz verbessern und die mit der Verstopfung verbundenen Symptome lindern.

Die typische Gewinnung von GOS umfasst die folgenden Schritte:
1. Konzentrierung einer Milchzuckerlösung (Lactose, Sauermolke, Milchpermeat mit dem Ziel eine Trockenmasse von mindestens 30 Gew.-% zu erreichen;
2. Hocherhitzung/UHT-Behandlung zur Entkeimung bei 85-140 C über 90 bis 300 s;
3. Zugabe des Enzyms (z.B. *Aspergillus Oryzae*) und Einstellung der für das Enzym optimalen pH- und Temperaturbedingungen (z.B. pH =4,5, 55 C);
4. Verweilzeit in der Regel über 30 bis maximal 120 min (abhängig vom Enzym), da ansonsten eine Rückspaltung erfolgt;
5. Thermische Inaktivierung des Enzyms beispielsweise durch Hochtemperaturpasteurisierung (90 °C, 10 min)
6. Aufreinigung, gegebenenfalls Konzentrierung, Sprühtrocknung.

Eine Übersicht zur Herstellung von Galactooligosacchariden findet sich auch von *K. Zerge* in einer Online-Publikation der Universität Dresden:
https://nbn-resolving.org/urn:nbn:de:bsz:14-qucosa-157121

### RELEVANTER STAND DER TECHNIK

EP 2620506 B1 (DUPONT) betrifft die Gewinnung von GOS ausgehend von Lactitol.

CN 104 004 799 B betrifft ein Verfahren zur kontinuierlichen Herstellung von Galactooligosacchariden, wobei 300-400 g/L einer Lactoselösung als Rohmaterial verwendet wird und Galactooligosaccharide direkt unter Verwendung von β-Galactosidase synthetisiert wird und der Galactooligosaccharidgehalt in der Reaktionslösung 40 Gew.-% oder mehr beträgt. Wenn die Laktosekonzentration 150 g/L oder weniger erreicht wird, wird die Reaktionslösung durch eine Ultrafiltrationsmembran ultrafiltriert, um das Enzym zu recyceln.

EP 3598901 B1 (HOCHSCHULE ANHALT) betrifft ein Verfahren zur Herstellung von GOS, bei dem man eine von L.bulgaricus (L.delbrueckii spp.bulgaricus) abgeleitete beta-Galactosidase bei einer Temperatur von 37 C oder weniger mit einer Lactose-haltigen Zusammensetzung wie Milch, Puffer oder Molke, z.B. Süßmolke, Sauermolke, Molkenkonzentrat oder Molkenpermeat, inkubiert.

EP 3041945 B1 (FRIESLAND) stellt ein Verfahren zur Herstellung von GOS aus Lactose bereit, das (i) das Inkontaktbringen einer Lactosebeschickung mit immobilisierter beta-Galactosidase (EC 3.2.1.23) und (ii) das Ermöglichen der GOS-Synthese umfasst, wobei die Lactosebeschickung eine wässrige Aufschlämmung von kristalliner Lactose ist.

WO 2008 037839 A1 (VALIO) bezieht sich auf ein Verfahren zur Herstellung von GOS-haltigen Produkten auf Milchbasis durch Behandlung mit einer beta-Galactosidase.

WO 2018 048305 A1 (UNIV GRONINGEN) beschreibt die Verwendung einer GOS-Zusammensetzung, die verzweigte und lineare GOS-Spezies mit einem Polymerisationsgrad (DP) von 3 umfasst, wobei die verzweigten DP3-GOS-Spezies im Überschuss gegenüber den linearen DP3-GOS-Spezies vorhanden sind, zur Induktion von Mucin-Glykan-Verwertungswegen in nützlichen Darmbakterien in einem Tier.

WO 2018 210820 A1 (NOVOZYMES) beansprucht ein Verfahren, bei dem Milchsubstrat mit einem Laktosegehalt von mindestens 20 Gew.-% Laktose wird mit einem Enzym mit transgalaktosylierender Aktivität behandelt wird. Die transgalaktosylierende Aktivität des Enzyms kann durch Glykation von Lysin- und/oder Argininresten durch Inkubation des Enzyms mit hohen Glukosekonzentrationen bei erhöhten Temperaturen erhöht worden sein.

WO 2020 049016 A1 (FRIESLAND) bezieht sich auf das Gebiet der hypoallergenen Oligosaccharide zur Verwendung in Nahrungszusammensetzungen, insbesondere auf Oligosaccharide mit präbiotischen Eigenschaften. Es wird eine hypoallergene Oligosaccharid-Zusammensetzung bereitgestellt, die Galactooligosaccharide (GOS) umfasst, wobei (i) der Gehalt an Galactooligosacchariden (GOS) mindestens 40 Gew.-% der Gesamttrockensubstanz der Zusammensetzung beträgt; (ii) der Gehalt an Allolactose mindestens 10 Gew.-% der Gesamttrockensubstanz der Zusammensetzung beträgt; (iii) der Gehalt an 6'-GL mindestens 30 Gew.-% der Gesamt-GOS in der Zusammensetzung beträgt; und (iv) mindestens 0. 5 Gewichtsprozent des gesamten GOS einen Polymerisationsgrad (DP) von sechs oder mehr aufweisen.

WO 2020 117548 A1 (DUPONT) betrifft ein Verfahren zur Bereitstellung eines laktosearmen Produkts auf Milchbasis mit GOS-Fasern, bei dem ein Milchsubstrat mit Laktose mit einem transgalaktosylierenden Enzym behandelt wird, um GOS-Fasern und verbleibende Laktose bereitzustellen; Deaktivieren des transgalaktosylierenden Enzyms; Inkontaktbringen des Substrats auf Milchbasis mit GOS-Fasern mit einer Laktase, um die verbleibende Laktose abzubauen, um das laktosearme Produkt auf Milchbasis mit GOS-Fasern bereitzustellen, und Deaktivieren der Laktase.

WO 2020 141032 A1 (FRIESLAND) bezieht sich auf das Gebiet der Nahrungsmittelbestandteile, insbesondere auf wirtschaftlich attraktive Verfahren zur Herstellung von hypoallergenen Galactooligosacchariden (HA-GOS) und deren Verwendung in Nahrungs- und Futtermitteln. Es wird ein Verfahren zur Herstellung eines HA-GOS-Präparats bereitgestellt, das das Inkontaktbringen eines Lactose-Einsatzmaterials mit einer speziellen beta-Galactosidase (EC 3.2.1.23) umfasst, wobei das Lactose-Einsatzmaterial ein Käsemolkenpermeat (CWP) oder ein CWP ist, das mit Sialyllactose angereichert ist (SL-CWP).

### AUFGABE DER ERFINDUNG

Derzeitig bekannte Prozesse zur Herstellung von GOS verlaufen sowohl sauer als auch neutral jeweils batchweise, wobei das Enzym durch thermische Desaktivierung vollständig verloren geht. Wünschenswert wäre ein vorzugsweise wenigstens in Teilen kontinuierlicher Prozess, bei dem das Enzym rückgewonnen und weiterverwertet werden kann .

### BESCHREIBUNG DER ERFINDUNG

Ein erster Gegenstand der Erfindung betrifft daher ein Verfahren zur Herstellung von Galactooligosacchariden, umfassend oder bestehend aus den folgenden Schritten:
(i) Bereitstellen einer wässrigen Milchzuckerlösung;
(ii) Entkeimung der Milchzuckerlösung;
(iii) Transgalactosilierung der in der entkeimten Milchzuckerlösung aus Schritt (ii) vorhandenen Milchzucker unter Zugabe mindestens einer beta-Galactosidase innerhalb deren optimalen Temperatur- und pH-Wert-Intervalls über einen Zeitraum von mindestens 30 Minuten unter Erhalt einer Reaktionsmischung;
(iv) Inhibierung der Enzymmasse in der Reaktionsmischung aus Schritt (iii) sowie
(v) Konfektionierung des Reaktionsmischung aus Schritt (iv),
wobei man
(a) die Enzymmasse durch Einstellen eines pH-Wertes außerhalb des Aktivitätsoptimums der Enzyme ganz oder teilweise inhibiert;
(b) die Reaktionsmischung zusammen mit der inhibierten Enzymmasse einer Filtration unter Erhalt eines Retentats (R1) und eines Permeats (P1) unterwirft;
(c) die inhibierte Enzymmenge als Retentat (R1) abtrennt und zum Schritt (iii) zurückführt; und
(d) das die Galactooligosaccharide enthaltende Permeat (P2) zum Schritt (v) weiterleitet.

Überraschenderweise wurde gefunden, dass das schnelle Herausführen der Reaktionslösung aus dem für die dort wirkenden Enzyme optimalen pH-Bereich eine effiziente, schnelle und technische einfache Maßnahme darstellt, um die enzymatische Reaktion abzubrechen und die Rückspaltung der gebildeten Oligomeren zu verhindern. Während die bisher angewandte Methode zur Inaktivierung der Enzyme, nämlich eine Ultrahocherhitzung über wenige Sekunden, dazu führt, dass das Enzymmaterial abgetötet wird, lassen sich die nach dem erfindungsgemäßen Verfahren inaktivierten Enzyme durch Rückführung in ihren optimalen pH-Wert-Bereich weiterverwenden. Dazu bedarf es lediglich, die Enzymmasse zu separieren und in den kontinuierlichen Prozess zurückzuführen. Natürlich erfolgt die Abtrennung und Rückführung nicht immer zu 100 %. Daher empfiehlt es sich, Verluste an Enzymmasse durch Dosierung von frischem Enzymmaterial kontinuierlich auszugleichen, so dass stets eine zumindest annähernd konstante Enzymmenge zur Verfügung steht. Dabei bedeutet "annähernd" ein Intervall von ± 5 Gew.-%.

Die Kombination aus pH-Wert-Änderung - nachfolgend als "pH-Shift" bezeichnet - und Abtrennung/Wiederverwertung der eingesetzten Enzyme, löst die eingangs geschilderte Aufgabe vollumfänglich. Insbesondere wird die Ökonomie des Verfahrens verbessert und damit der Preis für die Endprodukte vermindert, da Enzyme nicht länger vollständig verloren gehen. Zudem ist auf diese Weise bezogen auf die Abtrennung und Rückführung der Enzyme eine kontinuierliche Fahrweise möglich. Davon unabhängig kann das Verfahren natürlich auch in der beschriebenen Weise vollständig batchweise - beispielsweise in großen Fermentern - durchgeführt werden.

### Einsatzstoffe

Als Einsatzstoffe für die Herstellung von Galactooligosacchariden kommen Milchzuckerlösungen Frage, wie beispielsweise Lactoselösung, Sauermolke oder Milchpermeat. Wesentlich und gemeinsam ist den geeigneten Einsatzstoffen, dass sie eine ausreichende Menge an Lactose, speziell an glykosidisch gebundener Galactose besitzen.

Lactose, Milchzucker oder Laktose ist ein in Milch enthaltener Zucker.

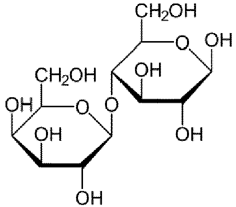

Das Disaccharid besteht aus den beiden Molekülen D-Galactose und D-Glucose, die über eine β-1,4-glycosidische Bindung miteinander verbunden sind. Nach IUPAC wird Lactose als 4-O-(β-D-Galactopyranosyl)-D-glucopyranose bezeichnet. Sie kommt als Hauptenergieträger in der Milch der Säugetiere vor. Lactose wird im Dünndarm vom Enzym Lactase verdaut, d. h. in Glucose und Galactose gespalten. In der Milch der Säugetiere sowie in Milcherzeugnissen macht Lactose fast den gesamten Anteil der Kohlenhydrate aus. Lactose, liefert Energie, unterstützt die Calcium-Resorption, hemmt Fäulnisbakterien im Darm des Menschen und begünstigt Bifidus-Bakterien (Bifidobacterium)

Molke ist die wässrige grünlich-gelbe Restflüssigkeit, die bei der Käseherstellung entsteht. Sie ist der flüssige Teil, der nach der Gerinnung der Milch zu Käse oder Quark abgesondert werden kann. Sauermolke entsteht, wenn Milch mit Milchsäurebakterien behandelt wird.

Milchpermeate insbesondere auf Basis von Magermilch sind seit langem bekannt. Es handelt sich um wässrige Flüssigkeiten, die bei der Filtration anfallen und bei der dem Einsatzprodukt Eiweiß und Milchfett entzogen wird. Zurück bleiben Vitamine, Mineralstoffe und Lactose. Für die Gewinnung von Milchpermeaten eignen sich grundsätzlich alle bekannten Filtrationsverfahren, als da sind: Diafiltration, Mikrofiltration, Ultrafiltration, Nanofiltration, Umkehrosmose, Elektrodialyse oder eine Kombination dieser Schritte.

Es empfiehlt sich, Milchzuckerlösungen mit einer ausreichend hohen Feststoffmenge (synonym: Trockenmasse) einzusetzen, um das erfindungsgemäße Verfahren mit ökonomisch sinnvollen Umsätzen und Ausbeuten durchführen zu können. Hierzu eignen sich Lösungen, die eine Feststoffmenge von etwa 25 bis etwa 50 Gew.-% und vorzugsweise etwa 30 bis etwa 35 Gew.-% aufweisen. Gegebenenfalls können technische Milchzuckerlösungen beispielsweise durch Umkehrosmose ("reverse osmosis RO") entsprechend aufkonzentriert werden.

### Entkeimung

Im ersten Schritt des erfindungsgemäßen Verfahrens werden die wässrigen Milchzuckerlösungen entkeimt. Darunter ist jeder Prozess zu verstehen, mit dem sich die Keimlast des natürlichen Ausgangsproduktes auf einen Wert vermindern lässt, der unter dem liegt, den die jeweiligen nationalen Prüfstellen als Schwelle für die Zulassung als Lebensmittel festgelegt haben. In der Regel werden die Milchzuckerlösungen auf unter 1.000 Keime/Liter entkeimt, vorzugsweise auf unter 500 Keime/Liter und insbesondere etwa 10 bis etwa 50 Keime/Liter. Die bevorzugte Entkeimungsmethode ist eine Hochtemperaturbehandlung, bei der die Lösungen einer Temperatur im Bereich von etwa 70 bis etwa 150 °C, vorzugsweise etwa 90 bis etwa 120 C über etwa 3 bis etwa 300 Sekunden, vorzugsweise etwa 50 bis etwa 200 Sekunden ausgesetzt werden. Eine Entkeimung kann entfallen, wenn bereits eine entkeimte Milchzuckerlösung eingesetzt wird.

### Enzymatische Transgalactosilierung

Die entkeimten Produkte werden im zweiten Verfahrensschritt einer enzymatischen Transgalactosilierung unterworfen. Hierunter versteht man die Übertragung von Galactoseeinheiten unter Aufbau eines oligomeren Zuckers in Gegenwart geeigneter Enzyme, in diesem Fall von beta-Galactosidasen, wobei Enzyme aus *Aspergillus oryzae, Bacillus circulans* oder Mischungen von beiden gemeinsam bzw. nacheinander zum Einsatz kommen.

Aspergillus oryzae, richtiger *Aspergillus flavus var. Oryzae* ist ein Schimmelpilz (Gießkannenschimmel), der in der japanischen Küche eine große Rolle spielt. Er ist der wichtigste unter den Köji-Pilzen Er wird vor allem benutzt, um Soja in Feststoff-Bioreaktoren zu fermentieren und so Miso und Sojasauce zu erzeugen.

*Bacillus circulans* ist eine Bakterienspezies, die sich zirkulär auf Nährmedien ausbreitet, woher auch ihr Namen stammt. Es handelt sich um anaerob wachsende, Gram variable stäbchenförmige, bewegliche Zellen, die 0,5 bis 1 µm breit und 3,5 µm lang sind. Das Bakterium fermentiert Pentosen, Hexosen, Hexitole und Disaccharide. *Bacillus circulans* kommt im Darm von pflanzenfressenden Fischen vor und unterstützt dort durch die Ausscheidung von Cellulasen die Verdauung.

Wie alle Enzyme weisen auch die beta-Galactosidasen ein vergleichsweise enges Temperatur- und pH-Wertintervall auf, in dem sie ihre optimale Leistung entfalten; diese sind dem Fachmann notorisch bekannt.

Mit *Aspergillus oryzae* wird die Reaktion daher vorzugsweise bei einer Temperatur im Bereich von etwa 50 bis etwa 60 °C und einem pH-Wert von etwa 4 bis etwa 5, bei Zugabe von Enzymen ex *Bacillus circulans* bei einer Temperatur im Bereich von etwa 45 bis etwa 55 °C und einem pH-Wert von etwa 5,5 bis etwa 6,5 durchführt. Bei Einsatz von Aspergillus oryzae wird daher auch von einem "sauren Verfahren", bei Bacillus circulans von einem "neutralen Verfahren" gesprochen.

Eine Besonderheit bei der grundsätzlichen Bildung von Galactooligosacchariden besteht darin, dass sich der Kettenaufbau nicht stetig fortsetzt, sondern nach einer Zeit an Geschwindigkeit verliert, bis sogar die Konkurrenzreaktion, nämlich die Rückspaltung der GOS überwiegt. Es hat sich daher als vorteilhaft erwiesen, der enzymabhängigen Reaktionskinetik Rechnung zu tragen und die Transgalactosilierung über einen Zeitraum von etwa 30 bis etwa 120 min und insbesondere von etwa 60 bis etwa 90 min durchzuführen.

### pH-Shift

Die Transgalactosilierung wird vorzugsweise solange durchgeführt, bis die höchste GOS-Konzentration erreicht ist. Dieser durch Enzym und Reaktionsbedingungen bedingte Wert kann durch Probennahme verfolgt und damit für den Fachmann leicht ermittelt werden. Ist das Maximum der GOS-Bildung erreicht, muss die Aktivität der Enzyme sehr schnell gestoppt werden, um eine Rückspaltung zu vermeiden. Dies erfolgte bisher durch schnelle Hocherhitzung, bei der allerdings das Enzymmaterial vollständig abgetötet wurde. Die vorliegende Erfindung geht einen anderen Weg und führt die Enzyme aus ihren optimalen Reaktionsbedingungen, konkret wird der pH-Wert durch Zugabe von Basen gegenüber dem Optimum um mindestens zwei Einheiten heraufgesetzt oder durch Zugabe von Säuren um mindestens zwei Einheiten herabgesetzt. Dieser pH-Shift bringt die Reaktion zwar nicht schlagartig zum Erliegen, vermindert die Aktivität der Enzyme jedoch um 80 bis 90 %, was für die Anforderungen ausreicht, eine nennenswerte Rückspaltung zu verhindern. Hierzu reicht es, den pH auf Werte von mindestens 7, vorzugsweise 8 bis 12 und insbesondere 9 bis 10 zu erhöhen oder auf Werte von 3 bis 5, vorzugsweise 1 bis 2 herabzusetzen. Bei der Änderung des pH-Wertes ist zu berücksichtigen, dass die Enzyme nicht irreversibel inaktiviert werden und der entsprechende pH-Wert - speziell im sauren Bereich - eine spätere Nutzung des Produktes nicht behindert. Der pH-Shift kann durch Zugabe einer erforderlichen Menge üblicher anorganischer Basen, wie beispielsweise einer wässrigen NaOH, durch Mineralsäuren wie HCI oder organische Säuren, wie beispielsweise Milchsäure erfolgen. Eine Anhebung des pH-Wertes ist gegenüber einer Absenkung bevorzugt.

### Abtrennung und Wiederverwendung der Enzyme

Die Abtrennung der inhibierten Enzyme erfolgt vorzugsweise durch Filtration und insbesondere durch Ultrafiltration, die ebenfalls vorzugsweise kontinuierlich durchgeführt wird. Die abgetrennte Enzymmenge wird wieder in den Reaktionskreislauf zurückgeführt und gelangt auf diese Weise wiederum direkt in den Temperatur-und pH-Wertbereich, in dem ihr Optimum sich befindet. Gegebenenfalls kann eine solche Menge frisches Enzym nachdosiert werden, so dass die Enzymaktivität über das kontinuierliche Verfahren hinweg konstant oder wenigstens annähernd konstant bleibt.

### Konfektionierung: Aufreinigung, Konzentrierung und Trocknung

Um konfektionierfähige Produkte zu erhalten, wird das Permeat aus dem vorangegangenen Schritt getrocknet und zuvor gegebenenfalls aufgereinigt und/oder aufkonzentriert.

In einer besonderen Ausführungsform des erfindungsgemäßen Verfahrens kann das Permeat einer Nanofiltration unterworfen werden, bei der unerwünschte Monosaccharide in das Permeat gehen und die Menge an GOS im Retentat angereichert wird. Alternativ können die Monosaccharide auch mit Hefen versetzt werden, die Lactose zu Ethanol und Kohlendioxid verstoffwechseln. Die Hefen können anschließend durch beispielsweise in einem Separator oder Dekanter mit nachfolgender Filtration abgetrennt und in den Kreislauf zurückgeführt werden. Schließlich ist es ebenso möglich, den restgehalt an Lactose durch Zugabe von Lactase zu vermindern.

Um die GOS-Konzentration zu erhöhen können die Permeate noch aufgereinigt werden, beispielsweise durch Elektrodialyse oder Membranverfahren, wie z.B. eine Umkehrosmose. Falls erforderlich, kann die Trockenmasse durch Eindampfen erhöht werden.

Die Trocknung erfolgt beispielsweise durch Lyophilisierung, vorzugsweise aber durch Sprühtrocknung, wobei die Temperatur im Einlass typischerweise etwa 180 bis etwa 260 °C und am Ausgang etwa 80 bis etwa 105 °C beträgt. Der Restwassergehalt beträgt dabei maximal 5 Gew.-% und vorzugsweise etwa 1 bis etwa 2 Gew.-%.

### BEISPIELE

### Beispiel 1

### Herstellung von GOS nach dem neutralen Verfahren ausgehend von Lactoselösung

1.000 kg einer 30 Gew.-%igen Lactoselösung wurde in einem Röhrenwärmeaustauscher über 120 Sekunden auf 98 C erhitzt und dabei entkeimt. Die entkeimte Lösung wurde auf 55 C abgekühlt, in einen Fermenter überführt, mit Hilfe von Milchsäure auf einen pH-Wert von 4,5 eingestellt, mit beta-Galactosidase *aus Bacillus circulans* im Gewichtsverhältnis Enzym:Substrat von 1:50 versetzt und gerührt. Der Fortschritt der Transgalactosilierung wurde durch Probennahme verfolgt. Nach etwa 90 Minuten war die maximale GOS-Konzentration erreicht. Der pH-Wert wurde durch Zugabe von 30 Gew.-%iger Natronlauge innerhalb von wenigen Minuten auf 10 erhöht, wodurch die Aktivität des Enzyms schlagartig um 80 % verringert wurde. Der Reaktionsansatz wurde zu einer Ultrafiltrationseinheit geleitet, die mit einer Spiralwickelmembran mit einer Porengröße von 10 kDa versehen war. Das inaktivierte Enzymmaterial wurde mit dem Retentat R1 in den Fermenter zurückgeführt; um Verlust auszugleichen wurden bezogen auf die Ausgangsmenge 5 Gew.-% frisches Enzym zugefügt. Das Permeat P1 wurde zu einer Nanofiltrationseinheit geleitet, die mit einer Keramikmembran mit einer Porenweite von 1.000 Da versehen war. Mit dem Permeat P2 wurden die noch im Produkt enthaltenen Monosaccharide abgetrennt, während das Retentat R2 einer Umkehrosmoseeinheit zugeleitet wurde, die mit einem Konzentrierungsfaktor von 1:2 arbeitete. Das dabei anfallende Permeat P3 (also das Konzentrierungswasser) wurde in das Verfahren zurückgeleitet, das Retentat R3 (also das GOS-Konzentrat) wurde im Plattenwärmeaustauscher 30 Sekunden auf etwa 85 C erhitzt und über einen Turm versprüht. Es wurde ein weißes Pulver mit einem GOS-Gehalt von mehr als 75 Gew.-% erhalten, welches noch eine Restfeuchte von 1 Gew.-% aufwies. Der DP der GOS betrug etwa 5 Gew.-%..

### Beispiel 2

### Herstellung von GOS nach dem sauren Verfahren ausgehend von Sauermolke

1.000 kg einer ca. 30 Gew.-%igen Sauermolke (pH = 5,1) wurde in einem Röhrenwärmeaustauscher über 120 Sekunden auf 98 C erhitzt und dabei entkeimt. Die entkeimte Lösung wurde auf 50 C abgekühlt, in einen Fermenter überführt, mit beta-Galactosidase aus *Aspergillus oryzae* im Gewichtsverhältnis Enzym:Substrat von 1:50 versetzt und gerührt. Der Fortschritt der Transgalactosilierung wurde durch Probennahme verfolgt. Nach etwa 90 Minuten war die maximale GOS-Konzentration erreicht. Der pH-Wert wurde durch Zugabe von 30 Gew.-%iger Natronlauge innerhalb von wenigen Minuten auf 10,0 eingestellt, wodurch die Aktivität des Enzyms schlagartig um 80 % verringert wurde. Der Reaktionsansatz wurde zu einer Ultrafiltrationseinheit geleitet, die mit einer Spiralwickelmembran mit einer Porengröße von 10 kDa versehen war. Das inaktivierte Enzymmaterial wurde mit dem Retentat R1 in den Fermenter zurückgeführt; um Verlust auszugleichen wurden bezogen auf die Ausgangsmenge 5 Gew.-% frisches Enzym zugefügt. Das Permeat P1 wurde mit 0,1 Gew.-% der Hefe *Kluyveromyces lactis* versetzt und weitere 5 h bei 30 bis 35 C gerührt. Anschließend wurde die Suspension einer zweiten Ultrafiltrationseinheit zugeführt, die Hefemasse als Retentat R2 abgetrennt und zurückgeführt und das Permeat P2 einer Umkehrosmoseeinheit zugeleitet, die mit einem Konzentrierungsfaktor von 1:10 arbeitete. Das dabei anfallende Permeat P3 (also das Konzentrierungswasser) wurde in das Verfahren zurückgeleitet, das Retentat R3 (also das GOS-Konzentrat) wurde im Plattenwärmeaustauscher auf etwa 200 C erhitzt und über einen Turm versprüht. Es wurde ein weißes Pulver mit einem GOS-Gehalt von mehr als 90 Gew.-% erhalten, welches noch eine Restfeuchte von 1 Gew.-% aufwies. Der DP der GOS betrug etwa 5 Gew.-%.

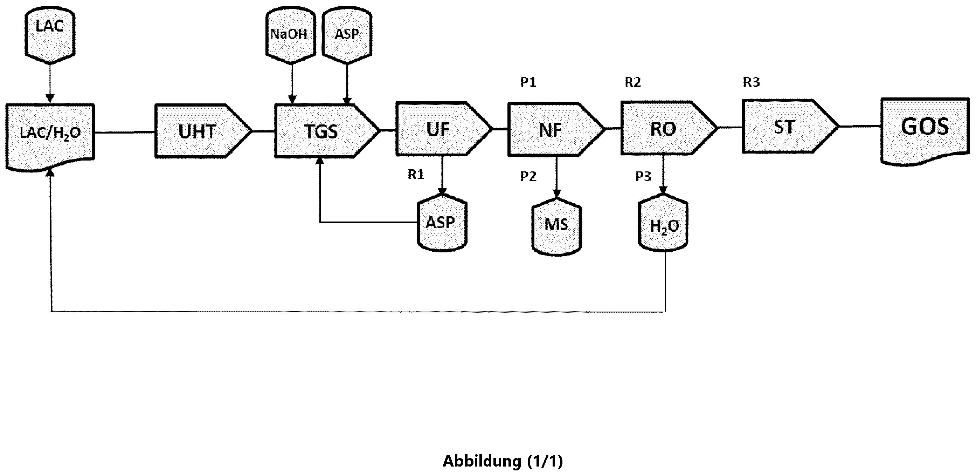

Beispiel 1 wird ferner an Hand eines Fließschemas gemäß **Abbildung 1** näher erläutert; dabei bedeuten die Abkürzungen:
- LAC: : Lactose
- LAC/H2O: : wässrige Lactoselösung
- UHT: : Ultrahocherhitzung
- TGS: : Transgalactosilierung
- ASP: : Aspergillus oryzae
- UF: : Ultrafiltration
- NF: : Nanofiltration
- MS: : Monosaccharide
- RO: : Umkehrosmose
- ST: : Sprühtrockung
- GOS: : Galactooliogosaccharide

## Patentansprüche

1. Verfahren zur Herstellung von Galactooligosacchariden, umfassend die folgenden Schritte:
(i) Bereitstellen einer wässrigen Milchzuckerlösung;
(ii) Entkeimung der Milchzuckerlösung;
(iii) Transgalactosilierung der in der entkeimten Milchzuckerlösung aus Schritt (ii) vorhandenen Milchzucker unter Zugabe mindestens einer beta-Galactosidase innerhalb deren optimalen Temperatur- und pH-Wert-Intervalls über einen Zeitraum von mindestens 30 Minuten unter Erhalt einer Reaktionsmischung;
(iv) Inhibierung der Enzymmasse in der Reaktionsmischung aus Schritt (iii) sowie
(v) Konfektionierung des Reaktionsmischung aus Schritt (iv),
wobei man
(a) die Enzymmasse durch Einstellen eines pH-Wertes außerhalb des Aktivitätsoptimums ganz oder teilweise inhibiert;
(b) die Reaktionsmischung zusammen mit der inhibierten Enzymmasse einer Filtration unter Erhalt eines Retentats (R1) und eines Permeats (P1) unterwirft;
(c) die inhibierte Enzymmenge als Retentat (R1) abtrennt und zum Schritt (iii) zurückführt; und
(d) das die Galactooligosaccharide enthaltende Permeat (P2) zum Schritt (v) weiterleitet.

2. Verfahren nach Anspruch 1, **dadurch gekennzeichnet, dass** man als Milchzuckerlösung eine wässrige Lactoselösung, Sauermolke oder Milchpermeat einsetzt.

3. Verfahren nach einem der Ansprüche 1 und/oder 2, **dadurch gekennzeichnet, dass** man Milchzuckerlösungen einsetzt, die eine Feststoffmenge von etwa 25 bis etwa 50 Gew.-% aufweisen.

4. Verfahren nach mindestens einem der Ansprüche 1 bis 3, **dadurch gekennzeichnet, dass** man die Entkeimung durch eine Hochtemperaturbehandlung bewirkt.

5. Verfahren nach Anspruch 4, **dadurch gekennzeichnet, dass** man die Hochtemperaturbehandlung bei einer Temperatur im Bereich von etwa 70 bis etwa 150 °C und über etwa 3 bis etwa 300 Sekunden durchführt.

6. Verfahren nach mindestens einem der Ansprüche 1 bis 5, **dadurch gekennzeichnet, dass** man als beta-Galactosidase Enzyme aus *Aspergillus oryzae* und/oder *Bacillus circulans* einsetzt.

7. Verfahren nach Anspruch 6, **dadurch gekennzeichnet, dass** man die Transgalactosilierung durch Zugabe von beta-Galactosidasen aus *Aspergillus oryzae* bei einer Temperatur im Bereich von etwa 50 bis etwa 60 °C und einem pH-Wert von etwa 4 bis etwa 5 durchführt.

8. Verfahren nach Anspruch 6, **dadurch gekennzeichnet, dass** man die Transgalactosilierung durch Zugabe von beta-Galactosidasen aus *Bacillus circulans* bei einer Temperatur im Bereich von etwa 45 bis etwa 55 °C und einem pH-Wert von etwa 5,5 bis etwa 6,5 durchführt.

9. Verfahren nach mindestens einem der Ansprüche 1 bis 8, **dadurch gekennzeichnet, dass** man die Transgalactosilierung über einen Zeitraum von etwa 60 bis etwa 600 min durchführt.

10. Verfahren nach mindestens einem der Ansprüche 1 bis 9, **dadurch gekennzeichnet, dass** man den pH-Wert durch Zugabe einer Base oder einer Säure einstellt.

11. Verfahren nach mindestens einem der Ansprüche 1 bis 10, **dadurch gekennzeichnet, dass** man die inaktivierte Enzymmasse durch Ultrafiltration abtrennt.

12. Verfahren nach mindestens einem der Ansprüche 1 bis 11, **dadurch gekennzeichnet, dass** man das Permeat P1 einer Nanofiltration unterwirft, die enthaltenen Monosaccharide als Permeat P2 abtrennt und das aufgereinigte Retentat R2 weiterverarbeitet.

13. Verfahren nach mindestens einem der Ansprüche 1 bis 12, **dadurch gekennzeichnet, dass** man das Permeat P1 mit einer Lactose verstoffwechselnden Hefe behandelt, die Hefe nach der Behandlung abtrennt und das Filtrat weiterverarbeitet.

14. Verfahren nach mindestens einem der Ansprüche 1 bis 13, **dadurch gekennzeichnet, dass** man das Transgalactosilierungsprodukt vor der Trocknung aufkonzentriert.

15. Verfahren nach mindestens einem der Ansprüche 1 bis 14, **dadurch gekennzeichnet, dass** man das Transgalactosilierungsprodukt einer Sprühtrocknung unterwirft.

## Claims

1. A process for the production of galactooligosaccharides, comprising the following steps:
(i) providing an aqueous lactose solution;
(ii) Sterilising the lactose solution;
(iii) Transgalactosylating the lactose present in the sterilised lactose solution from step (ii) by adding at least one beta-galactosidase within its optimal temperature and pH range over a period of at least 30 minutes to obtain a reaction mixture;
(iv) inhibiting the enzyme mass in the reaction mixture from step (iii) and;
(v) packaging the reaction mixture from step (iv),
wherein
(a) the enzyme mass is wholly or partially inhibited by adjusting the pH to a value outside the activity optimum;
(b) subjecting the reaction mixture, together with the inhibited enzyme mass, to filtration to obtain a retentate (R1) and a permeate (P1);
(c) separating the inhibited enzyme mass as retentate (R1) and returning it to step (iii); and
(d) sending the permeate (P2) containing the galactooligosaccharides on to step (v).

2. The process according to claim 1, **characterised in that** an aqueous lactose solution, acid whey or milk permeate is used as the lactose solution.

3. The process according to any one of claims 1 and/or 2, **characterised in that** milk sugar solutions are used which have a solids content of from about 25 to about 50 % by weight.

4. The process according to at least one of claims 1 to 3, **characterised in that** sterilisation is effected by high-temperature treatment.

5. The process according to claim 4, **characterised in that** the high-temperature treatment is carried out at a temperature in the range of about 70 to about 150 °C and for a duration of about 3 to about 300 seconds.

6. The process according to at least one of claims 1 to 5, **characterised in that** beta-galactosidase enzymes from Aspergillus oryzae and/or Bacillus circulans are used.

7. The process according to claim 6, **characterised in that** the transgalactosylation is carried out by adding beta-galactosidases from Aspergillus oryzae at a temperature in the range of about 50 to about 60 °C and a pH of about 4 to about 5.

8. The process according to claim 6, **characterised in that** the transgalactosylation is carried out by adding beta-galactosidases from Bacillus circulans at a temperature in the range of about 45 to about 55 °C and a pH of about 5.5 to about 6.5.

9. The process according to at least one of claims 1 to 8, **characterised in that** the transgalactosylation is carried out over a period of from about 60 to about 600 minutes.

10. The process according to at least one of claims 1 to 9, **characterised in that** the pH is adjusted by adding a base or an acid.

11. The process according to at least one of claims 1 to 10, **characterised in that** the inactivated enzyme mass is separated by ultrafiltration.

12. The process according to at least one of claims 1 to 11, **characterised in that** the permeate P1 is subjected to nanofiltration, the monosaccharides contained therein are separated as permeate P2, and the purified retentate R2 is further processed.

13. The process according to at least one of claims 1 to 12, **characterised in that** the permeate P1 is treated with a lactose-metabolising yeast, the yeast is separated off after treatment, and the filtrate is further processed.

14. The process according to at least one of claims 1 to 13, **characterised in that** the transgalactosylation product is concentrated prior to drying.

15. The process according to at least one of claims 1 to 14, **characterised in that** the transgalactosylation product is subjected to spray drying.

## Revendications

1. Procédé de préparation de galactooligosaccharides, comprenant les étapes suivantes :
(i) préparation d'une solution aqueuse de lactose ;
(ii) stérilisation de la solution aqueuse de lactose ;
(iii) transgalactosylation du lactose présent dans la solution stérilisée de l'étape (ii) par addition d'au moins une bêta-galactosidase dans la plage optimale de température et de pH de celle-ci, pendant une durée d'au moins 30 minutes, pour obtenir un mélange réactionnel ;
(iv) inhibition de la masse enzymatique dans le mélange réactionnel de l'étape (iii) ainsi que
(v) conditionnement du mélange réactionnel de l'étape (iv),
dans lequel on
(a) inhibe totalement ou partiellement la masse enzymatique en ajustant le pH en dehors de la plage d'activité optimale ;
(b) soumettre le mélange réactionnel, conjointement avec la masse enzymatique inhibée, à une filtration pour obtenir un rétentat (R1) et un perméat (P1) ;
(c) séparer la quantité d'enzyme inhibée sous forme de rétentat (R1) et la renvoyer à l'étape (iii) ; et
(d) on achemine le perméat (P2) contenant les galactooligosaccharides vers l'étape (v).

2. Le procédé selon la revendication 1, **caractérisé en ce qu'**on utilise comme solution de lactose une solution aqueuse de lactose, du petit-lait acide ou un perméat de lait.

3. Le procédé selon l'une des revendications 1 et/ou 2, **caractérisé en ce que** l'on utilise des solutions de lactose présentant une teneur en matières sèches d'environ 25 à environ 50 % en poids.

4. Le procédé selon au moins l'une des revendications 1 à 3, **caractérisé en ce que** la stérilisation est réalisée par un traitement à haute température.

5. Le procédé selon la revendication 4, **caractérisé en ce que** le traitement à haute température est effectué à une température comprise entre environ 70 et environ 150 °C et pendant une durée comprise entre environ 3 et environ 300 secondes.

6. Le procédé selon au moins l'une des revendications 1 à 5, **caractérisé en ce que** l'on utilise, comme bêta-galactosidases, des enzymes issues d'Aspergillus oryzae et/ou de Bacillus circulans.

7. Le procédé selon la revendication 6, **caractérisé en ce que** l'on effectue la transgalactosylation par addition de bêta-galactosidases issues d'Aspergillus oryzae à une température comprise entre environ 50 et environ 60 °C et à un pH compris entre environ 4 et environ 5.

8. Le procédé selon la revendication 6, **caractérisé en ce que** l'on effectue la transgalactosylation par addition de bêta-galactosidases issues de Bacillus circulans à une température comprise entre environ 45 et environ 55 °C et à un pH compris entre environ 5,5 et environ 6,5.

9. Le procédé selon au moins l'une des revendications 1 à 8, **caractérisé en ce que** l'on effectue la transgalactosylation pendant une durée comprise entre environ 60 et environ 600 minutes.

10. Le procédé selon au moins l'une des revendications 1 à 9, **caractérisé en ce que** l'on ajuste le pH par addition d'une base ou d'un acide.

11. Le procédé selon au moins l'une des revendications 1 à 10, **caractérisé en ce que** l'on sépare la masse enzymatique inactivée par ultrafiltration.

12. Le procédé selon au moins l'une des revendications 1 à 11, **caractérisé en ce que** l'on soumet le perméat P1 à une nanofiltration, on sépare les monosaccharides contenus sous forme de perméat P2 et on traite ultérieurement le rétentat purifié R2.

13. Le procédé selon au moins l'une des revendications 1 à 12, **caractérisé en ce que** l'on traite le perméat P1 avec une levure métabolisant le lactose, on sépare la levure après le traitement et on traite ensuite le filtrat.

14. Le procédé selon au moins l'une des revendications 1 à 13, **caractérisé en ce que** l'on concentre le produit de transgalactosylation avant le séchage.

15. Le procédé selon au moins l'une des revendications 1 à 14, **caractérisé en ce que** l'on soumet le produit de transgalactosylation à un séchage par pulvérisation.
